## Europäisches Patentamt

## European Patent Office

## Office européen des brevets

(19)

(11) Publication number: **0 003 380**
**B1**

(12) **EUROPEAN PATENT SPECIFICATION**

(45) Date of publication of patent specification: **27.01.82**

(51) Int. Cl.³: **C 07 C 43/29, C 07 C 41/22**

(21) Application number: **79200031.7**

(22) Date of filing: **17.01.79**

(54) Process for the manufacture of a mixture of 3-phenoxybenzyl bromide and 3-phenoxybenzal bromide.

(30) Priority: **30.01.78 US 873310**

(43) Date of publication of application:
**08.08.79 Bulletin 79/16**

(45) Publication of the grant of the European patent:
**27.01.82 Bulletin 82/4**

(84) Designated Contracting States:
**BE CH DE FR GB NL**

(56) References cited:
**FR - A - 2 305 419**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Smutny, Edgar Josef**
**1701 Maryland Street**
**Houston Texas 77006 (US)**
Inventor: **Colby, Thomas Harold**
**2622 Pomeran Drive**
**Houston Texas 77080 (US)**
Inventor: **Ryder, Elliott Elkington, Jr.**
**1008 Black Wood Place**
**Modesto California 95355 (US)**

(74) Representative: **Keuzenkamp, Abraham et al,**
**P.O. Box 302**
**NL-2501 CH Den Haag (NL)**

Courier Press, Leamington Spa, England.

Process for the manufacture of a mixture of 3-phenoxybenzyl bromide and 3-phenoxybenzal bromide

This invention relates to a process for the manufacture of a mixture of 3-phenoxybenzyl bromide and 3-phenoxybenzal bromide which mixture is useful as an intermediate in the manufacture of pyrethroid insecticides.

It is known from Netherlands Patent Application 7701128 that a mixture of 3-phenoxybenzyl bromide and 3-phenoxybenzal bromide can be used as a precursor for the preparation of insecticidal esters of certain cyclopropanecarboxylic acids: it also discloses a process for preparing these insecticidal esters from such a mixture of bromides. In the procedures by which the mixtures of bromides are converted to the insecticidal esters, it has been found very desirable that the mixture of bromides produced be substantially free from nuclear brominated by-products, for these by-products, primarily 6-bromo-3-phenoxytoluene and 6-bromo-3-phenoxybenzyl bromide, or by-products derived therefrom appear as undesirable contaminants in the insecticidal ester product. Further, it has been found that higher apparent yields of insecticidal product are obtained from benzal bromide than from benzyl bromide and hence it is desirable that the ratio, benzal bromide/benzyl bromide, be as high as possible.

It is also known from Netherlands patent application 7701128 that a mixture of 3-phenoxybenzyl bromide and 3-phenoxybenzal bromide can be prepared by the direct bromination of 3-phenoxytoluene in the presence of a free radical initiator, such as ultra-violet light. It is further known from French Patent Specification 2.305.419 that 3-phenoxybenzyl bromide can be prepared by reacting 3-phenoxytoluene with bromine in the presence of ultra-violet light at an elevated temperature.

It has been found that the known route to this mixture of bromides can be improved by modifying the reaction conditions such that the nuclear brominated by-products are minimised and the starting 3-phenoxytoluene is substantially converted into bromides.

Accordingly, the invention provides a process for the manufacture of a mixture of 3-phenoxybenzyl bromide and 3-phenoxybenzal bromide by the reaction of a molar excess of molecular bromine on 3-phenoxytoluene, characterized in that the molar quantity of bromine is in the range 1.3 to 1.5 mole of bromine per mol. of the toluene and in that the reaction is carried out at a temperature in the range 195° to 235°C in the presence of an inert gas and in the absence of (a) actinic radiation, (b) an added catalyst or initiator, and (c) an added solvent.

The process according to the invention converts 3-phenoxytoluene to the benzyl- and benzal bromides, with minimum nuclear bromination, minimum production of other undesirable by-products and minimum content of the 3-phenoxytoluene in the end product. Important features in the process are:—

(a) the temperature at which the bromine is brought into contact with the 3-phenoxytoluene. As indicated above, this temperature lies within the range 195°C to 235°C, preferably within the range 200°C to 230°C, with optimum conversion to the desired product being attained at a temperature of about 225°C. Any contact, even transient local contact, between the 3-phenoxytoluene and the bromine at a temperature outside of this range is preferably avoided. Contact of the reactants at a lower temperature results in increased nuclear bromination; contact at a higher temperature results in increased production of other by-products, known as "heavy ends", i.e., higher boiling materials of undetermined identity. Thus, the 3-phenoxytoluene itself initially should preferably be at a temperature within the indicated range, and more preferably the bromine is also at a temperature within or near the indicated range when it is brought into contact with the 3-phenoxytoluene, and the bromine is fed at a controlled rate, with effective, intimate mixing, the mixture being held within the indicated range throughout the mixing of the two reactants;

(b) the amount of bromine used, relative to the amount of the 3-phenoxytoluene used. As pointed out hereinbefore, this amount lies within the range 1.3 to 1.5 moles per mole of the 3-phenoxytoluene, with optimum conversion of the 3-phenoxytoluene to the desired product being effected when about 1.4 moles of bromine are used per mol. of the 3-phenoxytoluene. Moreover, when this amount of bromine is used, the ratio, benzal bromide/benzyl bromide is highest. When the amount of bromine present exceeds about 1.5 moles per mol. of the 3-phenoxytoluene, the amount of "heavy ends" increases;

(c) the presence of an inert gas when the bromine is brought into contact with the 3-phenoxytoluene.

Also, the formation of undesirable by-products is further minimized by ensuring that no local excess of bromine relative to the 3-phenoxytoluene occurs. This can be achieved by thorough intimate mixing of the bromine with the 3-phenoxytoluene and inert gas, and by controlling the rate at which the bromine is introduced into the 3-phenoxytoluene.

The bromine preferably is brought into contact with the 3-phenoxytoluene at a controlled rate which is essentially the rate at which it reacts with the 3-phenoxytoluene. The bromine reacts very quickly, so that one way of determining the maximum rate at which it can be added is to monitor the by-product hydrogen bromide off-gas, the presence of molecular bromine therein indicating that the bromine is being added too rapidly. In the usual situation, with adequate mixing, the bromine addition time is of the order of four hours or somewhat longer, with a period of four to six hours apparently being preferable.

Contact between the bromine and the 3-phenoxytoluene must be effected in the presence of a gas which is inert in the reaction zone. While unreactive gaseous inorganic compounds (such as carbon dioxide) or organic compounds (such as carbon tetrachloride) can be used, preferably an inert gaseous element, such as nitrogen, neon, argon or the like, is to be preferred. Being readily available at a relatively low price, nitrogen ordinarily will be found to be quite acceptable.

The amount of the inert gas needed is not known to be critical. Thus to ameliorate the high reactivity of the 3-phenoxytoluene and the bromine, a significant amount of the gas is preferably present in the reaction mixture, but too high a concentration of nitrogen in the reaction mixture will tend to interfere with the progress of the reaction, and will require an unnecessarily large reactor and associated vapour handling equipment. It appears most appropriate to relate the amount of the inert gas to the amount of bromine used. On this basis it appears that from about one-half mole to about four moles of inert gas per mole of bromine is suitable, with from about 0.75 to about 2 moles of inert gas per mol. of bromine being most suitable.

The inert gas is most effectively and conveniently introduced into the reaction mixture by mixing it with the bromine vapour before it is introduced into the reaction zone.

Hydrogen bromide is formed as a by-product of the bromination of the 3-phenoxytoluene. To avoid possible undesirable side reactions the hydrogen bromide is removed from the reaction mixture essentially as quickly as it is formed. This removal is ordinarily effected automatically, since the hydrogen bromide will be swept from the reaction zone by the inert gas as it passes therethrough.

When the bromination of the 3-phenoxytoluene is carried out as described above, essentially all of the bromine is consumed, with none appearing in the inert gas/hydrogen bromide off-gas and little or none being present in the final reaction mixture.

Any unreacted bromine, and any hydrogen bromide, present in the final mixture can be removed therefrom by continuing to pass the inert gas through the mixture after all of the bromine has been added. To avoid any undesirable side reaction, the temperature of the reaction mixture should be maintained within the indicated range during this treatment.

The final reaction mixture is suitable without further purification as a starting material in the preparation of pyrethroid insecticides.

The treatment of the 3-phenoxytoluene with the bromine is conveniently carried out at atmospheric pressure — or in the usual case, the slightly elevated autogenous pressure resulting from passage of the gases into and out of the reactor and associated equipment. However, operation under higher pressure is quite feasible, provided adequate precautions, as already described, are taken to avoid possible nuclear bromination by the bromine and hydrogen bromide present.

It is to be noted that the bromination of 3-phenoxytoluene, according to this invention, is conducted in the dark, without using light or other actinic radiation; without the use of an added catalyst or initiator and without the use of an added inert solvent.

The process of the invention is described in the following Examples, which are included herein to illustrate the conduct of the process.

Examples I and II

In these Examples, bromination of 3-phenoxytoluene was conducted in a four-litre vessel equipped with a heating mantle. Stirring was effected by a six-bladed turbine. Bromine was dripped into a dry flask heated to about 250°C, dry nitrogen being passed through the flask, carrying the bromine vapour into the reaction vessel, and carrying by-product hydrogen bromide out of the vessel. The nitrogen flow rate through the flask was 3.47 moles/hour, standard temperature and pressure.

The results of the experiments are reported in Tables 1 and 2. In the Tables the following abbreviations are used:

POT: 3-phenoxytoluene
POMB: 3-phenoxybenzyl bromide
PODB: 3-phenoxybenzal bromide
POBT: 6-bromo-3-phenoxytoluene
POBB: 6-bromo-3-phenoxybenzyl bromide

In the experiments reported in Table 1, aliquot samples of the reaction mixture were taken from time to time as the reaction progressed. It will be noted that of the 14 runs, runs 7 and 14 are according to the invention and the remaining runs are included for comparative purposes; run 7 is Example I and run 14 is Example II.

3

TABLE 1

| Temperature, (°C) | Run | Moles bromine / moles POT | Composition of product (%w) [c] | | | | | | Moles POMB + PODB / Moles POT charged | Moles POT converted / Moles POT charged |
|---|---|---|---|---|---|---|---|---|---|---|
| | | | POT | POMB | PODB | POBT | POBB | Heavy ends | | |
| 225 | 1 | 0.246 | 70.8 | 29.2 | 1.2 | 0.2 | — | 0.2[a] | 0.231 | 0.217 |
| | 2 | 0.490 | 45.6 | 49.6 | 3.8 | 0.3 | — | 0.6[a] | 0.446 | 0.447 |
| | 3 | 0.728 | 26.5 | 60.1 | 8.6 | 0.5 | 0.1 | 1.3[a] | 0.615 | 0.651 |
| | 4 | 0.954 | 14.3 | 68.2 | 15.5 | 0.5 | 0.2 | 2.3[a] | 0.793 | 0.794 |
| | 5 | 1.162 | 5.5 | 67.6 | 23.6 | 0.4 | 0.3 | 3.7 | 0.903 | 0.918 |
| | 6 | 1.290 | 2.4 | 62.0 | 31.0 | 0.2 | 1.2 | 4.5 | 0.937 | 0.962 |
| (Example I) | 7* | 1.412 | 0.9 | 53.3 | 40.0 | 0.1 | 1.1 | 5.9 | 0.949 | 0.985 |
| 200 | 8 | 0.249 | 72.3 | 27.0 | 1.6 | 0.7 | — | 0.1[b] | 0.219 | 0.199 |
| | 9 | 0.479 | 47.2 | 44.2 | 6.1 | 1.3 | 0.2 | 0.3[b] | 0.413 | 0.429 |
| | 10 | 0.710 | 29.8 | 53.7 | 12.4 | 1.5 | 0.4 | 0.7[b] | 0.519 | 0.610 |
| | 11 | 0.938 | 17.3 | 57.7 | 20.3 | 1.5 | 1.2 | 1.1[b] | 0.723 | 0.757 |
| | 12 | 1.156 | 8.6 | 53.4 | 29.2 | 1.4 | 2.0 | 1.6[b] | 0.798 | 0.871 |
| | 13 | 1.282 | 5.3 | 51.3 | 37.3 | 1.2 | 2.5 | 2.0 | 0.871 | 0.917 |
| (Example II) | 14* | 1.414 | 2.8 | 45.2 | 44.3 | 1.0 | 2.8 | 2.5 | 0.898 | 0.955 |

[a] Estimated from samples 5, 6 and 7.

[b] Estimated from samples 13 and 14.

[c] Variation from 100% total due to analytical methods used.

* process according to the invention.

Examples III to VI

Further experiments were conducted to ascertain conditions for minimizing formation of the 6-bromo-3-phenoxytoluene, without reducing yield of the desired benzyl and benzal bromides. A reaction temperature of 225°C was used. The results are summarized in Table 2.

TABLE 2

| | Example | | | |
|---|---|---|---|---|
| | III[a] | IV | V | VI |
| Charge | | | | |
| POT, g | 2200 | 2400 | 2400 | 2400 |
| Br$_2$, g | 2570 | 2942 | 2932 | 2931 |
| Br$_2$/POT (mol./mol.) | 1.41 | 1.41 | 1.40 | 1.40 |
| N$_2$/Br$_2$ (mol./mol.) | 1.10 | 1.04 | 0.85 | 0.85 |
| Reaction time, hrs | 5.1 | 5.5 | 4.5 | 4.5 |
| Product | | | | |
| Weight, g | 3164[b] | 3684[c] | 3741[c] | 3768[c] |
| Analysis, %w | | | | |
| POT | 0.9 | 1.0 | 1.0 | 1.0 |
| POMB | 53.3 | 50.9 | 52.3 | 52.6 |
| PODB | 40.0 | 40.8 | 39.4 | 38.3 |
| POBT | 0.1 | 0.2 | 0.2 | 0.1 |
| POBB | 1.1 | 1.6 | 1.7 | 1.6 |
| Heavy ends | 5.9[d] | 6.5[e] | 5.4[e] | 7.4[e] |
| Recovered yield, %m [f] | 84.6[g] | 88.4 | 90.1 | 90.2 |

[a] This Example used in study of bromination quantities adjusted for periodic samples taken.

[b] 3373 grams expected, basis POT and Br$_2$ charge.

[c] 3855 grams expected, basis POT and Br$_2$ charge.

[d] Determined by distillation.

[e] Determined from gas chromatography closure.

[f] Basis moles POMB + PODB/mol. POT charged.

[g] Low recovered yield due to substantial mechanical loss during sampling.

It has also been established that

(a) as the reaction temperature is raised, production of heavy ends increases;
(b) as the reaction temperature is lowered, the amount of 6-bromo-3-phenoxytoluene increases;
(c) as the amount of bromine used is reduced, relative to the amount of 3-phenoxytoluene, the amount of unconverted 3-phenoxytoluene increases, and the amount of 3-phenoxybenzal bromide decreases;

5

(d) as the amount of bromine used is increased, relative to the amount of 3-phenoxytoluene, the amount of unreacted 3-phenoxytoluene in the product decreases, and the ratio of 3-phenoxybenzal bromide/3-phenoxybenzyl bromide increases, representing a net gain in useful product. As the amount of bromine is further increased, the amount of heavy ends increases, resulting in a net loss of useful product.

## Claims

1. A process for the manufacture of a mixture of 3-phenoxybenzyl bromide and 3-phenoxybenzal bromide by the reaction of a molar excess of molecular bromine on 3-phenoxytoluene, characterized in that the molar quantity of bromine is in the range 1.3 to 1.5 moles of molecular bromine per mol. of the toluene, and in that the reaction is carried out at a temperature in the range 195°C to 235°C in the presence of an inert gas and in the absence of: (a) actinic radiation; (b) an added catalyst or initiator; and (c) an added solvent.

2. A process according to claim 1, characterized in that the temperature used is in the range 200°C to 230°C.

3. A process according to claim 1 or 2, characterized in that there is used about 1.4 moles of bromine per mol. of the 3-phenoxytoluene.

4. A process according to any one of the preceding claims, characterized in that intimate mixing of the bromine, the 3-phenoxytoluene and the inert gas is achieved.

## Patentansprüche

1. Verfahren zur Herstellung eines Gemisches aus 3-Phenoxybenzyl-bromid und 3-Phenoxybenzalbromid durch Umsetzen eines molaren Überschusses von molekularem Brom mit 3-Phenoxytoluol, dadurch gekennzeichnet, dass die molare Menge an Brom im Bereich von 1,3 bis 1,5 Mol molekularem Brom je Mol des Toluols liegt und dass die Umsetzung bei einer Temperatur im Bereich von 195°C bis 235°C in Anwesenheit eines Inertgases und in Abwesenheit von (a) aktinischer Strahlung, (b) einem zugesetzten Katalysator oder Initiator und (c) einem zugesetzten Lösungsmittel durchgeführt wird.

2. Verfahren nach Anspruch 1, dadurch gekennzeichnet, dass die angewandte Temperatur im Bereich von 200°C bis 230°C liegt.

3. Verfahren nach Anspruch 1 oder 2, dadurch gekennzeichnet, dass etwa 1,4 Mol Brom je Mol 3-Phenoxytoluol verwendet werden.

4. Verfahren nach einem der vorangehenden Ansprüche, dadurch gekennzeichnet, dass das Brom, das 3-Phenoxytoluol und das Inertgas innig miteinander vermischt werden.

## Revendications

1. Procédé pour la préparation d'un mélange du bromure de 3-phénoxybenzyle et du bromure de 3-phénoxybenzylidène par la réaction d'un excès molaire de brome moléculaire sur le 3-phénoxytoluène, caractérisé en ce que la quantité molaire de brome est comprise entre 1,3 et 1,5 mole de brome moléculaire par mole du toluène, et en ce que la réaction est conduite à une température comprise entre 195°C et 235°C en présence d'un gaz inerte et en l'absence (a) de radiation actinique, (b) d'un catalyseur ou initiateur ajouté et (c) d'un solvant ajouté.

2. Procédé selon la revendication 1, caractérisé en ce que la température utilisée est comprise entre 200°C et 230°C.

3. Procédé selon l'une des revendications 1 et 2, caractérisé en ce qu'on utilise environ 1,4 mole de brome par mole du 3-phénoxytoluène.

4. Procédé selon l'une quelconque des revendications précédentes, caractérisé en ce qu'on effectue un mélange intime du brome, du 3-phénoxytoluène et du gaz inerte.

6